Europäisches Patentamt

European Patent Office

Office européen des brevets

(19)

(11) Publication number: **0 251 612 B1**

(12) **EUROPEAN PATENT SPECIFICATION**

(45) Date of publication of patent specification: **16.03.94**

(51) Int. Cl.5: **C12P 21/00**, C12N 15/00, G01N 33/569, A61K 39/42, G01N 33/577

(21) Application number: **87305505.7**

(22) Date of filing: **22.06.87**

The file contains technical information submitted after the application was filed and not included in this specification

(54) **Human monoclonal antibody to lymphadenopathy-associated virus.**

(30) Priority: **23.06.86 US 877579**

(43) Date of publication of application:
**07.01.88 Bulletin 88/01**

(45) Publication of the grant of the patent:
**16.03.94 Bulletin 94/11**

(84) Designated Contracting States:
**AT BE CH DE ES FR GB GR IT LI LU NL SE**

(56) References cited:
**EP-A- 0 185 444**
**EP-A- 0 201 716**
**EP-A- 0 214 709**
**WO-A-86/06414**
**FR-A- 2 580 177**

**Immunology Today vol.4,nber 3,1983,pp72-73**

(73) Proprietor: **Bristol-Myers Squibb Company**
**345 Park Avenue**
**New York, N.Y. 10154(US)**

(72) Inventor: **McClure, Janela**
**Route 1, Box 1096**
**Vashon Island Washington 98070(US)**

(74) Representative: **Jones, Alan John et al**
**CARPMAELS & RANSFORD**
**43 Bloomsbury Square**
**London, WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

Technical Field

The present invention relates generally to the use of immunological techniques to provide novel materials useful in diagnosing and treating viral infections as well as useful in biochemical and histological studies. More particularly, the present invention relates to the production and characterization of human monoclonal antibodies capable of reacting with the lymphadenopathy associated virus, LAV/HTLV-III.

Background Art

Acquired Immune Deficienty Syndrome (AIDS) is a transmissible deficiency of cellular immunity characterized by opportunistic infections and certain rare malignancies. The dominant risk groups for AIDS include homosexually active males, intravenous drug abusers, recipients of transfusions and blood products, and the heterosexual partners and children of high-risk individuals, suggesting the involvement of an infectious agent transmitted through intimate contact or blood products.

Recent evidence indicates that the infectious agent responsible for disease transmission is a novel lymphotropic retrovirus, known as Lymphadenopathy-Associated Virus (LAV) (Barre'-Sinoussi et al., Science 225: 840 (1984) and designated Human T-cell Lymphotropic Virus (HTLV-III), AIDS-Associated Retrovirus (ARV), Immune Deficiency-Associated Virus (IDAV), or human immunodeficiency virus (HIV). Still more recent data indicate that LAV, HTLV-III, ARV, and IDAV share several important characteristics, including substantial nucleotide homology (Wain-Hobson et al., Cell 40:9 (1985); Muesing et al., Nature 313:450 (1985); Sanchez-Pescador et al., Science 227:484 (1985), and should be considered isolates of the same virus, although there is a likelihood that strain-to-strain variations among the viral isolates will exist. In addition to exhibiting substantial nucleotide homology, the isolates are similar with respect to morphology, cytopathology, requirements for optimum reverse transcriptase activity, and at least some antigenic properties (Levy, Supra; Schupbach et al., Science 224:503 (1984).

The transmissibility of the AIDS virus through blood products (blood, blood serum, blood plasma, and fractions thereof) makes it important to screen the blood products to determine if donors have been exposed to the virus and are potential carriers. Several products that use disrupted viral antigen in ELISA formats are currently being marketed for this purpose. Individuals whose blood contains antibodies to LAV/HTLV-III are said to be "seropositive." Blood from these sero-positive donors may be eliminated from the blood supply upon detection, thereby helping to prevent the spread of the disease.

The lack of antibody response to LAV/HTLV-III is not always indicative of lack of infectivity of blood products. Virus has been cultured from blood samples collected from individuals found to be antibody negative and showing no signs of clinical manifestations (Salahuddin, S. Z., et al., Lancet ii:1418 (1984)). There is a need in the art for an antigen capture assay system to prevent these infective, antibody negative blood products from entering into the blood supply. Monoclonal antibodies reactive with major viral components may provide the basis for such a system of antigen detection.

Recently there has been a report of murine monoclonal antibodies reactive with the p24 core protein of LAV/HTLV-III and its protein precursors and a murine antibody reactive with the envelope glycoprotein gp41. (di Marzo Veronese et al. Proc. Natl. Acad. Sci. USA 82:5199 (1985); di Marzo Veronese et al., Science 229:1402 (1985). Chassagne et al. (J. Immunol. 136:1442 (1986)) have also described a murine monoclonal antibody specific for p24 and its precursors. These antibodies have been used experimentally to trace the processing of core protein precursors in infected cells to final viral proteins.

Although it is possible to detect antibody-positive individuals and may be possible to detect those antigen-positive, no effective treatment or preventative measure for the disease have yet been found. With the spread of AIDS beginning to reach extraordinary, if not epidemic proportions, and the extent to which the virus may be transmitted still in question, there is a need in the art for a composition with prophylactic and/or therapeutic effects.

Disclosure of Invention

Briefly stated, the present invention discloses (1) human monoclonal antibodies capable of reacting with an antigenic determinant of LAV/HTLV-III; (2) immortalized cell lines that produce these human monoclonal antibodies; and (3) methods for utilizing the human monoclonals, for instance, in determining the presence of LAV/HTLV-III in biological samples. Another method described herein includes a procedure for separating specific antigenic determinants of LAV/HTLV-III from a mixture. Further, pharmaceutical compositions are

EP 0 251 612 B1

disclosed which comprise a therapeutically effective amount of a human monoclonal antibody capable of reacting with an antigenic determinant of LAV/HTLV-III, and a physiologically acceptable carrier and/or diluent. Compositions having this characteristic are particularly useful within a method for significantly reducing the infectivity of LAV/HTLV-III in warm-blooded animals.

As noted above, one aspect of the present invention provides a method for determining the presence of LAV/HTLV-III in a biological sample. The method generally involves (a) incubating a human monoclonal antibody capable of reacting with an antigenic determinant of LAV/HTLV-III with a biological sample; and (b) detecting the presence of immunecomplexes formed between the monoclonal antibody and the biological sample, and therefrom determining the presence of LAV/HTLV-III. Labeled or unlabeled monoclonal antibodies may be used. The method is applicable to detecting LAV/HTLV-III in a wide variety of biological samples, including bodily secretions, bodily fluids, and tissue specimens.

Another aspect of the present invention, as noted above, provides a method for separating specific antigenic determinants of LAV/HTLV-III from a mixture containing antigenic determinants of LAV/HTLV-III. The method comprises (a) immobilizing a human monoclonal antibody capable of reacting with the specific antigenic determinants on a substrate; (b) contacting the mixture containing the LAV/HTLV-III antigenic determinants with the immobilized antibody under suitable conditions such that immunecomplexes are formed between the antibody and the specific antigenic determinants; and (c) separating the immunecomplexes from the mixture.

Other aspects of the invention will become evident upon reference to the following detailed description and attached drawings.

Brief Description of the Drawings

Figure 1 illustrates the origin of the LAV inserts in pENV-3.
Figure 2 illustrates the amino acid sequence of ENV-3.

Best Mode for Carrying Out the Invention

Prior to setting forth the invention, it may be helpful to an understanding thereof to set forth definitions of certain terms to be used hereinafter.

Lymphadenopathy-Associated Virus (LAV): A human T-lymphotropic retrovirus. For purposes of the present invention, a virus is considered to be the same as or equivalent to LAV if it substantially fulfills the following criteria:

(a) the virus is tropic for T-lymphocytes, especially T-helper cells (CD4+, according to the international nomenclature defined in Bernard et al., eds., Leucocyte Typing, New York: Springer Verlag (1984));

(b) the virus is cytopathic for infected CD4+ cells (rather than transforming, as are HTLV-I and II);

(c) the virus encodes an RNA-dependent DNA polymerase (reverse transcriptase) which is $Mg^{2+}$-dependent (optimum concentration 5 mM, optimum pH 7.8, not inhibitable by actinomycin D) and can employ $(dT)_{12-18}$ as a primer for reverse transcription from its $3'$ LTR;

(d) the virus bands in a sucrose gradient at a density of approximately 1.16;

(e) the virus can be labeled with $[^3H]$ uridine;

(f) the virus is distinct by immunological and nucleotide sequence criteria from members of the HTLV-I/II family of viruses (by this criterion HTLV-III is not to be considered a member of the HTLV-I/II family);

(g) the virus is substantially cross-reactive immunologically with proteins encoded by the gag and env regions of LAV; and

(h) the virus shares substantial nucleotide homology (78-100%) and amino acid sequence homology (90-100%) with LAV.

The Human Retrovirus Subcommittee of the International Committee on the Taxonomy of Viruses has recommended the designation of Human Immunodeficiency Virus (HIV) (Science 232:697 (1986)), therefore, for purposes of the present invention, LAV/HTLV-III and HIV are considered to be equivalent.

In accordance with the present invention, a novel hybrid cell is provided for the specific recognition of the proteins and protein precursors of the human immunodeficiency virus, LAV/HTLV-III. The subject cells have an identifiable chromosome, in which the germ line DNA has rearranged to encode an antibody having a binding site for an epitope common to some or all the human immunodeficiency virus clinical isolates, but not found on other human retroviruses, such as HTLV-I and HTLV-II. These human monoclonal antibodies can be used in a wide variety of ways, including diagnosis and therapy.

The preparation of monoclonal antibodies can be accomplished by immortalizing the expression of nucleic acid sequences that code for antibodies specific for an epitope on antigens of LAV/HTLV-III.

3

Typically the monoclonal antibodies are produced by cell-driven Epstein-Barr virus (EBV) transformation of B-lymphocyte cells obtained from human donors who are or have been exposed to LAV/HTLV-III. The antibody secreting cell lines so produced are characterized as continuously growing lymphoblastoid cells that possess a diploid karyotype, are Epstein-Barr nuclear antigen positive, and secrete monoclonal antibody of either IgG, IgM, IgA, or IgD isotype, including various subtypes such as IgG1, IgG2, IgG3 and IgG4. The cell-driven transformation process itself is described in detail in U.S. Patent No. 4,464,465, which is incorporated herein by reference. The monoclonal antibodies may be used intact, or as fragments, such as $F_v$,Fab, $F(ab')_2$, but usually intact.

Alternatively, cell lines producing the antibodies could be produced by cell fusion between suitably drug-marked human myeloma, mouse myeloma, or human lymphoblastoid cells with human B-lymphocytes to yield human hybrid cell lines.

The cell line of the present invention may find uses other than for the direct production of the human monoclonal antibodies. The cell line may be fused with other cells (such as suitably drug-marked human myeloma, mouse myeloma or human lymphoblastoid cells), to produce hybridomas, and thus provide for the transfer of genes encoding the monoclonal antibodies. Alternatively, the cell line may be used as a source of the chromosomes encoding the immunoglobulins, which may be isolated and transferred to cells by techniques other than fusion. In addition, the genes encoding the monoclonal antibodies may be isolated and used in accordance with recombinant DNA techniques for the production of the specific immunoglobulin in a variety of hosts. Particularly, by preparing cDNA libraries from messenger RNA, a single DNA clone, coding for the immunoglobulin and free of introns, may be isolated and placed into suitable prokaryotic or eukaryotic expression vectors and subsequently transformed into a host for ultimate bulk production.

The lymphoblastoid or hybrid cell lines may be cloned and screened in accordance with conventional techniques, and antibodies in the cell supernatants detected that are capable of binding to the LAV/HTLV-III viral proteins, recombinant fusion proteins, or synthetic peptides. The appropriate hybrid cell lines may then be expanded in vitro or injected into the peritoneal cavity of an appropriate host for production of ascites fluid. By virtue of having the antibody of the present invention, which is known to be specific for the LAV/HTLV-III virus, the supernatants may be screened in competition with the subject monoclonal antibodies in a competitive assay. Thus, hybrid cell lines can be readily produced from a variety of sources based on the availability of the present antibodies specific for the particular antigen.

The monoclonal antibodies of the present invention are particularly useful because of their specificity for gp41, expressed fusion proteins and synthetic antigens of LAV/HTLV-III from the envelope region.

The monoclonal antibodies can also find a wide variety of utilities in vitro. By way of example, the monoclonal antibodies can be utilized for assaying whether virus is present in infected cultured lymphocytes by indirect immunofluorescence. Viral proteins or portions of viral proteins can be removed from disrupted purified viral preparation or from complex mixtures of which these proteins are constituents.

These specific viral proteins can be removed by attachement of the monoclonal antibodies to a support material such as polymeric tubes, beads, polysaccharide particulates and the like. Methods of attachment are well known in the art, see for example Schall and Tenoso, Immunoassays: Clinical Laboratory Techniques for the 1980's: 127 (1980) Alan R. Liss Inc. Mixtures are combined with the immobilized antibody under conditions which will allow binding to occur. Immune-complexes are separated from the rest of the mixture by methods appropriate for the support. These methods are well known in the art. Isolated viral proteins can then be released from the antibody by use of conditions unfavorable to immune-complex formation.

For diagnostic purposes, the monoclonal antibodies may either be labeled or unlabeled. Typically, diagnostic assays entail the detection of the formation of a complex through the binding of the monoclonal antibody to the LAV/HTLV-III antigen. When unlabeled, the antibodies find use in agglutination assays. In addition, unlabeled antibodies can be used in combination with other labeled antibodies (second antibodies) that are reactive with the monoclonal antibody, such as antibodies specific for immunoglobulin. Alternatively, the monoclonal antibodies can be directly labeled. A wide variety of labels may be employed, such as radionuclides, fluorescers, enzymes, enzyme substrates, enzyme cofactors, enzyme inhibitors, ligands (particularly haptens), etc. Numerous types of immunoassays are available, and by way of example, some include those described in U.S. Patent Nos. 3,817,827; 3,850,752; 3,901,654; 3,935,074; 3,984,533; 3,996,345; 4,034,074; and 4,098,876, all of which are herein incorporated by reference.

Commonly, the monoclonal antibodies of the present invention are utilized in enzyme immunoassays, where the subject antibodies, or second antibodies from a different species, are conjugated to an enzyme. When a biological sample containing LAV/HTLV-III antigens, such as human blood serum or viral cell culture supernatant, is combined with the subject antibodies, binding occurs between the antibodies and

EP 0 251 612 B1

those molecules exhibiting the desired epitope. Such proteins or viral particles may then be separated from the unbound reagents, and a second antibody (labeled with an enzyme) added. Thereafter, the presence of the antibody-enzyme conjugate specifically bound to the antigen is determined. Other conventional techniques well known to those skilled in the art may also be utilized.

Kits can also be supplied for use with the subject antibodies in the detection of LAV/HTLV-III infection or for the presence of LAV/HTLV-III antigen. Thus, the subject monoclonal antibody composition of the present invention may be provided, usually in a lyophilized form, either alone or in conjunction with additional antibodies specific for other epitopes of LAV/HTLV-III. The antibodies, which may be conjugated to a label or unconjugated, are included in the kits with buffers, such as Tris, phosphate, carbonate, etc., stabilizers, biocides, inert proteins, e.g., bovine serum albumin, or the like. Generally, these materials will be present in less than about 5% wt. based on the amount of active antibody, and usually present in total amount of at least about 0.001% wt. based again on the antibody concentration. Frequently, it will be desirable to include an inert extender or excipient to dilute the active ingredients, where the excipient may be present in from about 1 to 99% wt. of the total composition. Where a second antibody capable of binding to the monoclonal antibody is employed, this will usually be present in a separate vial. The second antibody is typically conjugated to a label and formulated in an analogous manner with the antibody formulations described above.

As indicated previously, the detection of antigen is useful in diagnosing present infection by the LAV/HTLV-III virus. The presence of the virus in various biological samples can also be accomplished. Biological samples can include, but are not limited to, blood serum, saliva, semen, tissue biopsy samples (brain, skin, lymphnodes, spleen, etc.), cell culture supernatants, disrupted viral and bacterial expression systems and the like. Presence of virus is tested for by incubating the human monoclonal antibody with the biological sample under conditions conducive to immunecomplex formation, followed by the detection of complex formation. In one embodiment, complex formation is detected through use of a second antibody capable of binding to the monoclonal antibody which is typcially conjugated to a label and formulated in an analagous manner with the antibody formulations described above.

Those skilled in the art will realize that the monoclonal antibodies of the present invention will also find use in numerous additional ways, such as affinity chromatography, purification of various naturally occurring LAV/HTLV-III antigens, as well as expressed from various expression systems (i.e., E. coli, vaccinia, chinese hamster ovary cells, and others), histological staining reagents, and the like. See, generally, Immunological Methods, Vols. I and II, Eds. Lefkovits, I. and Pernis, V., Academic Press, New York (1979 and 1981); and Handbook of Experimental Immunology, Ed. Weir, D., Blackwell Scientific Publications, St. Louis, MO (1978), both of which are incorporated herein by reference.

Therapeutically, antibodies with proper biological properties are useful directly as therapeutic agents. Alternatively, the antibodies can be bound to a toxin to form an immunotoxin or a radioactive material or drug to form a radiopharmaceutical or pharmaceutical. Methods for producing immunotoxins and radiopharmaceuticals of antibodies are well known (see, for example, Cancer Treatment Reports 68:317 (1984)). The conjugated antibodies are mixed with physiologically acceptable carriers, such as sterile water, saline, buffered saline. Adjuvants can also be employed, such as aluminum hydroxide.

Other features and advantages of the present invention will become apparent from the following experimental descriptions, which describe the invention by way of example. This example is offered by way of illustration and not by way of limitation.

EXAMPLE

This example demonstrates methods for the production of human monoclonal antibodies that react with LAV viral proteins and characterization of those antibodies using synthetic peptides and bacterially expressed fusion proteins in immunoblots and enzyme-linked immunoassays.

A peripheral blood sample obtained from a healthy AIDS positive blood donor served as a source of human B cells. Mononuclear cells were separated from the blood by standard centrifugation techniques on Ficoll-Paque and washed twice in calcium/magnesium-free phosphate buffered saline (PBS).

The mononuclear cells were depleted of T-cells using a modified E-rosetting procedure. Briefly, the cells were first resuspended to a concentration of $1 \times 10^7$ cells/ml in PBS containing 20% fetal calf serum (FCS) at 4°C. One ml of this suspension was then placed in a 17 x 100 mm polystyrene round bottom tube to which was added $1 \times 10^9$ 2-amino-isothioronium bromide (AET)-treated sheep red blood cells from a 10% (v/v) solution in Iscove's modified Dulbecco's medium (Iscove's medium). The suspension was very gently mixed for 5-10 minutes at 4°C and the E-rosetted cells then removed by centrifugation on Ficoll-Paque for 8 minutes at 2500xg at 4°C. E-rosette negative peripheral blood mononuclear cells (E⁻PBMC)

5

banding at the interface were collected and washed once in Iscove's medium and resuspended in same containing 15% (v/v) FCS, L-glutamine (2mmol/l), penicillin (100 IU/ml), streptomycin (100 ug/ml), hypoxanthine (1 x $10^{-4}$ M), aminopterin (4 x $10^{-7}$ M), and thymidine (1.6 x $10^{-5}$ M). This medium is hereafter referred to as HAT medium.

Cell-driven transformation of the E⁻PBMC was accomplished by co-cultivating these cells with a transforming cell line. The transforming cell line was an Epstein-Barr nuclear antigen (EBNA) positive human lymphoblastoid cell line derived by ethyl methane-sulphonate (EMS) mutagenesis of the GM 1500 lymphoblastoid cell line followed by selection in the presence of 30 ug/ml 6-thioguanine to render the cells hypoxanthine-guanine phosphoribosyl transferase (HGPRT) deficient and thus HAT sensitive. This cell line is denominated by the 1A2 cell line and was deposited at the American Type Culture Collection (A.T.C.C.) on March 29, 1982, under A.T.C.C. No. CRL 8119. 1A2 cells in logarithmic growth phase were suspended in HAT medium and then combined with the E⁻PBMC. The cell mixture was plated into eight round-bottom 96-well microtiter plates (Costar 3799) at a concentration of 72,000 cells/well in a volume of 200 ul per well, and incubated at 37°C in a humidified atmosphere containing 6% $CO_2$. Cultures were fed on days 5 and 8 post-plating by replacement of half the supernatant with fresh HAT medium. The wells were observed every other day on an inverted microscope for signs of cell proliferation. Fourteen days post plating, it was observed that 100% of the wells contained proliferating cells and that by day 21 most of the wells contained cells of sufficient density for removal and testing of supernatants for anti-LAV antibody.

Supernatants were screened for the presence of anti-LAV antibody using standard ELISA technique.

Briefly, Immulon II plates (Dynatech) were coated with disrupted whole virus in carbonate/bicarbonate buffer pH 9.6 and incubated Overnight at 4°C. Rinsed with phosphate buffered saline 0.05% Tween 20 (PBS-Tween) and then blocked with Blotto (PBS, pH 7.2, containing 5% (w/v) non-fat dry milk, 0.01% (v/v) antifoam A (Sigma), and 0.01% (w/v) Thimerosal for 60 minutes at room temperature. The plates were then rinsed three times with PBS-Tween and allowed to dry. Supernatants from wells with growing clones were added to the coated, blocked plates and incubated at 37°C for 45 minutes, followed again by washing three times with PBS-Tween. Peroxidase-goat anti-human IgG (1:2,000 dilution in PBS-Tween, Antibodies Inc.), was added (10 ul/well). Incubation was for 45 minutes at 37°C and washed as above. Enzyme substrate, O-phenylenediamine and hydrogen peroxide, was added and plates were incubated for 30 minutes at room temperature in the dark. The reactions were stopped with 3N $H_2SO_4$ and quantitated using an automated microplate reader.

Analysis of the culture supernatants led to the identification of one well (LT1/41-H1) which contained anti-LAV antibodies. This supernatant was further characterized by immunoblot, immunoprecipitation and ELISA with fusion proteins. The antibodies from this clone were found to be reactive with gp41 by blot and ENV-3, peptide 39 and peptide 79 by ELISA. Cell line LT1/41-H1 has been deposited with the ATCC under Accession No. CRL-9128.

These antigens are important because they are from a region of LAV/HTLV-III which is consistently recognized by serum from antibody positive donors and is present throughout the course of the disease (Sarngadharan et al., Science 224:506 (1984); Safai et al., Lancet 1984-I, 1438 (1984).

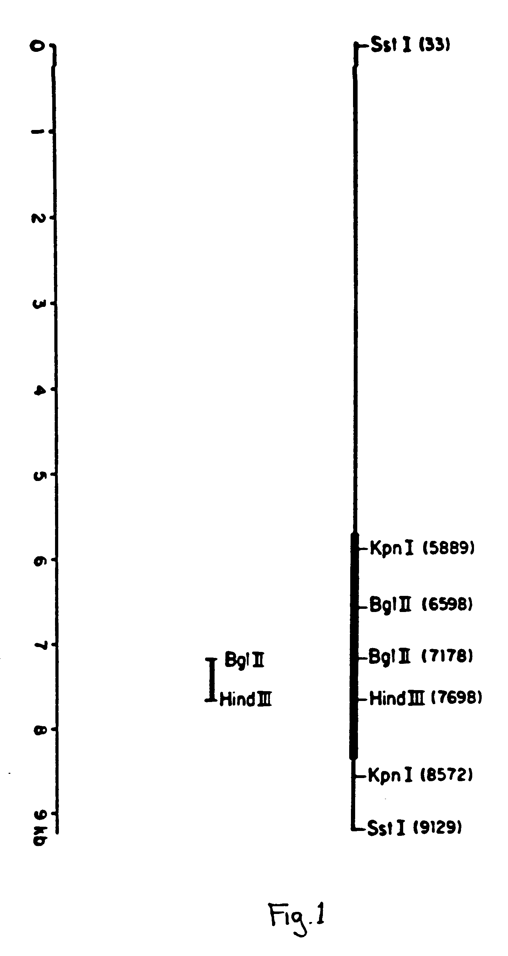

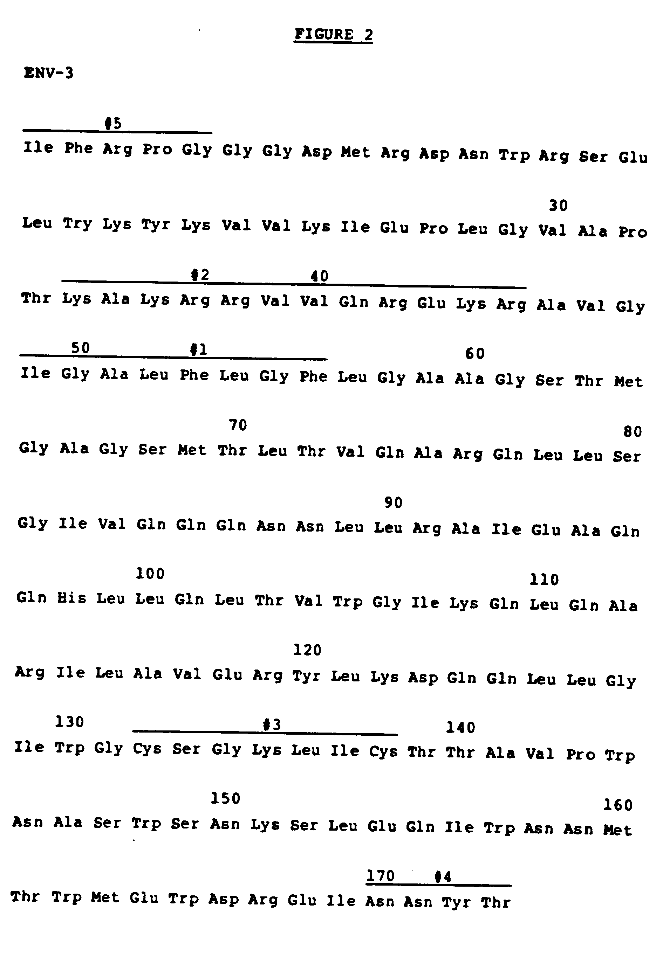

The protein ENV-3 is a bacterially expressed fusion protein from pENV-3 (ATCC accession #53072) which is a region of LAV from base pair 7178 to 7698 (numbering according to Wain-Hobson et al., Cell 44:9 (1985)) (Figures 1 and 2).

Peptide 39 is a synthetic polypeptide defined as encoding the region from about base pair 7516 through 7593 and has the following amino acid sequence, where oligopeptides included within the following sequence will include linear epitopes within such sequence:

ARG-ILE-LEU-ALA-VAL-GLU-ARG-TYR-LEU-LYS-
ASP-GLN-GLN-LEU-LEU-GLY-ILE-TRP-GLY-CYS-
SER-GLY-LYS-LEU-ILE-CYS-X, where X is OH or $NH_2$

Table 1 depicts a comparison of peptide 39 with whole virus lysate in an ELISA for the detection of antibodies to LAV/HTLV-III.

## TABLE 1

### COMPARISON OF PEPTIDE 39 WITH WHOLE VIRUS LYSATE
### IN AN ELISA ASSAY FOR THE DETECTION OF ANTIBODIES TO LAV

| Positive Sera | Diagnosis | ELISA Using Whole Virus Lysate[1] | Confirmed as Sero-positive[2] | Pep 39 |
|---|---|---|---|---|
| 155 | LAS[3] and/or homosexual | 1.069 | yes | 1.167 |
| 124 | LAS and/or homosexual | 1.189 | yes | 1.073 |
| 138 | LAS and/or | 1.302 | yes | 0.514 |
| 133 | LAS and/or homosexual | 1.250 | yes | 1.036 |
| 134 | LAS and/or homosexual | 1.050 | yes | 1.691 |
| 153 | LAS and/or homosexual | 2.000 | yes | 1.314 |
| 157 | LAS and/or homosexual | 1.349 | yes | 1.326 |
| Y-1/ | LAS and/or homosexual | 2.000 | yes | 1.305 |
| 501 | LAS and/or homosexual | 1.109 | yes | 1.167 |
| 1892 | Healthy heterosexual | n.d. | n.d.[4] | 0.045 |
| 639 | Healthy heterosexual | 0.123 | not seropositive | 0.038 |

[1] Prepared as described in U.K. application Serial No. 83/24800, filed September 15, 1983, (see EP-A-0 138 667).

[2] Radiolabeled LAV antigens were disrupted in RIPA buffer (Gilead et al., Nature (1976) 264:263) and then were reacted with human serum. The resultant immune complexes were separated by binding to a Staphylococcus aureus adsorbent (Kessler, J. Immunology (1975) 115:1617) followed by multiple washings. Immuneprecipitated antigens were analyzed by SDS polyacrylamide gel electrophoresis (Laemmli, Nature (1970) 227:680) followed by fluorography. Presence of either a p25 or gp43 band was considered

necessary and sufficient to confirm a sample as seropositive.

3 LAS = lymphadenopathy syndrome.

4 N.D. = not determined.

Peptide 79 is defined as encoding the region from about base pair 7543 through 7593 and includes any oligopeptides coding for linear epitopes within the following amino acid sequence:

**Y-LYS-ASP-GLN-GLN-LEU-LEU-GLY-ILE-TRP-GLY-**

**CYS-SER-GLY-LYS-LEU-ILE-CYS-X,**

wherein X is OH or $NH_2$ and Y is TYR or CYS.

Table 2 depicts the results of an ELISA for the detection of antibodies to LAV/HTLV-III using peptide 79.

**TABLE 2**
**PEPTIDE 79 IN AN ELISA ASSAY FOR THE**
**DETECTION OF ANTIBODIES TO LAV**

| Serum No. | Confirmed as Seropostive | 79 |
|---|---|---|
| 127 | yes | 2.346 |
| 130 | yes | 1.808 |
| 124 | yes | 1.086 |
| 125 | yes | 2.266 |
| 128 | yes | 1.144 |
| 134 | yes | 1.316 |
| 135 | yes | .381 |
| 153 | yes | 1.039 |
| 154 | yes | ND |
| 155 | yes | 1.584 |
| 157 | yes | 1.162 |
| 120 | yes | 1.546 |
| 121 | yes | 2.084 |
| 132 | yes | 1.386 |
| 138 | yes | .312 |
| 133 | yes | .597 |
| 131 | yes | 1.150 |
| 501 | yes | 1.768 |
| 129 | yes | .562 |
| Y1 | yes | ND |
| N3 | no | .224 |
| N12 | no | .174 |
| N4 | no | .172 |
| 639 | no | .153 |
| 641 | no | .140 |
| N13 | no | .226 |
| N14 | no | .162 |
| N16 | no | .183 |
| Cutoff | | 0.30 |
| Fraction of Confirmed Seropositive samples detected as positive | | 18/18 |

8

## Western Blotting

Characterization by Western immunoblotting was carried out on clone supernatants using purified LAV virus and recombinant fusion proteins as antigens. These antigens were first separated by gradient gel electrophoresis (7.0-15.0%) and transferred to nitrocellulose membrane (NCM) by electrophoresis for four hours at 25V in 25 mM sodium phosphate (pH 7.0). After transfer, the NCM was blocked in PBS-Tween for one hour at room temperature. The NCM was incubated with goat anti-human IgG-Horse radish peroxidase diluted in PBS-Tween for one hour at room temperature. This was followed by washing in PBS-Tween and then immersion in horse radish peroxidase color development solution (Bio-Rad Laboratories, Richmond, CA) for 20 minutes. The reaction was stopped by immersion in deionized water. Monoclonal antibody reactivity was compared to a standard positive serum reaction with purified disrupted virus run as a positive control.

## Immunoprecipitation

Viral extracts for radioimmune precipitation were prepared from CEM cells (ATCC #CCL119) infected with the LAV 1 isolate of LAV/HTLV-III adapted to lytic growth by continuous passage in tissue culture. When early cytopathic effect was evident, the cells were transferred to labeling media containing $^{35}$[S] methionine (50 uCi/ml) or $^{3}$[H] glucosamine (25 uCi/ml), then incubated for 24 h until most of the cells had lysed, releasing virus into the culture supernatant. Virus was pelleted (one hour at 100,000 xg) from the cell-free culture supernatant, and detergent extracts were prepared in P-RIPA buffer (phosphate buffered saline containing 1.0% Triton X-100, 1.0% dedxycholic acid, 0.1% SDS, and 1.0% aprotinin). Similar extracts were prepared from uninfected cells after washing once in PBS. Immunoprecipitation assays were performed with 100 ul extract volume incubated with culture supernatant for 1 h on ice. Immunoprecipitin (100 ul; BRL) resuspended in P-RIPA containing 1.0% ovalbumin, was added to each tube and incubated for an additional 30 minutes. The bound complexes were washed and separated by SDS-PAGE (7.0-15.0% gradient gel). Following electrophoresis, the cells were fixed, soaked in Enhance (NEN), dried, and exposed to Kodak EX-5 film.

A reference positive serum which immuno-precipitated all LAV/HTLV-III viral protein was reacted with $^{35}$[S]methionine-labeled extract from culture supernatant of mock-infected cells and with $^{35}$[S]methionine-labeled viral extract as a positive and negative control.

## Enzyme-Linked Immunosorbent Assay

The antibody LT1/41-H1 was further characterized by ELISA. Methods are as described above except bacterially expressed fusion proteins and synthetic peptides are used in place of disrupted whole virus as antigen.

## Indirect Immunofluorescence Assay

Indirect immunofluorescence assays were carried out on acetone fixed and live cells. Acetone fixed slides prepared from LAV-infected CEM cells were incubated with culture supernatant for one hour at 37°C while live cells were incubated with culture supernatant for one hour at 4°C before the cells were plated and fixed. In both methods, reactive cells were detected with fluorescein isothiocyanate-labeled anti-human IgG (Antibodies Inc.).

Neutralization assays were carried out on the monoclonal antibody by serial dilution of virus into 100 ul of antibody supernatant or control serum. A set of 1:5 dilutions of virus was prepared using a 96 well microtiter plate. Tested with the LT1/41-H1 antibody were heat inactivated LAV seropositive serum with known neutralizing activity (1:10 dilution in medium), heat inactivated normal human serum (1:10 dilution in medium), supernatant from an irrelevant human monoclonal antibody producing cell line, and a medium control. These preparations were filtered with 0.45 u filter before use. A viral concentration of approximately $2.5 \times 10^4$ tissue culture infective does (TCID) was used to start the serial dilution. Antibody and virus were incubated for one hour at 37°C. A second 96 well plate was prepared with CEM-F cells plated at $1 \times 10^5$ cells/well in 150 ul, followed by inoculation with 50 ul/well (about $1 \times 10^4$ TCID in first column) of the antibody-virus mixture. The second plate was incubated at 37°C for 24-48 hours, at which time all of the supernatant was removed. Fresh medium (200 ul) was added and the plate was incubated for an additional 7-14 days with refeeding every 24-48 hours. On days 7, 10, and 14 the cells are harvested and assayed for virus expression by immunofluorescence.

Cloning of specific antibody producing cells from well LT1/41-H1 was accomplished by subjecting the cells to several rounds of limiting dilution cloning until all clonal supernatants assayed by the above methods resulted in antibodies which were able to immunoblot and immunoprecipitate gp41 and gave positive reactions with pENV-3, peptide 39 and peptide 79 by ELISA. Cloning employed feeder cells as described above for subculturing. By these means, a cloned transformed human cell line was achieved which is continuous (immortal) and which secretes a human monoclonal antibody with the characteristics described above. In this example, the cell line and the antibody it produces carry the same designation.

Neutralization can be carried out by an alternate method. Culture supernatant was used after five-fold concentration with an Amicon-Centricon microconcentrator or unconcentrated. A dilution series was constructed with an initial 1:5 dilution followed by a two-fold dilution series using 25 ul of concentrated or unconcentrated culture supernatant diluted in medium (RPMI 1640, 15% fetal calf serum, 40% heat inactivated human serum). Virus ($1\times10^5$-$1\times10^6$ tissue culture infectious units/ml) diluted 1:400 or 1:600 in medium was added to the constructed dilution series to a total volume of 50 ul. Samples were incubated for 45 minutes at 37°C in a humidified chamber.

Infectivity was tested by the ability of the treated virus to infect MT-2 cells, an HTLV-I carrying cell line highly susceptible to LAV/HTLV-III infection. The MT-2 cells were plated in Costar flat bottom 96 well plate at $1\times10^5$ cells/well in 100 ul of medium. The plate was incubated for 45 minutes at 37°C before the addition of 20 ul of the treated virus was added to each well. After 5-7 days the MT-2 cells were examined for syncytia formation which is indicative of LAV/HTLV-III infection.

**Claims**
**Claims for the following Contracting States : AT, BE, CH, DE, FR, GB, IT, LI, LU, NL, SE**

1. A human monoclonal antibody capable of reacting with an antigenic determinant of LAV/HTLV-III, wherein the human monoclonal antibody blocks the binding of an antibody produced by cell line LT1/41-H1 to said antigenic determinant.

2. A human monoclonal antibody that binds an epitope on the envelope glycoprotein gp41.

3. The human monoclonal antibody of claim 2 wherein the epitope is defined by the polypeptide encoded by pENV-3.

4. The human monoclonal antibody of claim 2 wherein the epitope is defined by peptide 39.

5. The human monoclonal antibody of claim 2 wherein the epitope is defined by peptide 79.

6. An immortalized cell line that produces human monoclonal antibody capable of reacting with an antigenic determinant of LAV/HTLV-III, wherein the cell line is a hybrid of human B-lymphocytes capable of producing antibodies to an antigenic determinant of LAV/HTLV-III, and a cell selected from the group consisting of human myleoma cells, mouse myleoma cells, and human lymphoblastoid cells.

7. An immortalized cell line that produces human monoclonal antibody capable of reacting with an antigenic determinant of LAV/HTLV-III, wherein the cell line comprises B-lymphocyte cells capable of producing antibodies to an antigenic determinant of LAV/HTLV-III, transformed with Epstein-Barr virus transformed cells.

8. The cell line LT1/41-H1 (ATCC CRL-9128).

9. A monoclonal antibody produced by the cell line of claim 8.

10. The human monoclonal antibody of any one of Claims 1 to 5 or 9 tagged with a label capable of providing a detectable signal.

11. A method for determining the presence of LAV/HTLV-III in a biological sample comprising:
    incubating a human monoclonal antibody capable of reacting with an antigenic determinant of LAV/HTLV-III with a biological sample; and
    detecting the presence of immunecomplexes formed between said monoclonal antibody and said biological sample, and therefrom determining the presence of LAV/HTLV-III.

12. The method of claim 11 wherein said monoclonal antibody blocks the binding of an antibody produced by cell line LT1/41-H1 to said antigenic determinant.

13. The method of claim 11 wherein the monoclonal antibody binds an epitope on the envelope glycoprotein gp41.

14. The method of claim 11 wherein the monoclonal antibody is produced by the cell line LT1/41-H1.

15. The method of any one of Claims 11 to 14 wherein the monoclonal antibody is labeled.

16. The method of claim 15 wherein said label is selected from the group consisting of radionuclides, fluorescers, enzymes, enzyme substrates, enzyme cofactors, enzyme inhibitors, and ligands.

17. The method of any one of Claims 11 to 16 wherein the step of detection is by enzyme reaction, fluorescence, radioactivity, cell lysis, or luminescence emission.

18. The method of any one of Claims 11 to 17 wherein the biological sample is selected from the group consisting of bodily secretions, bodily fluids, and tissue specimens.

19. A pharmaceutical composition comprising a therapeutically effective amount of a human monoclonal antibody capable of reacting with an antigenic determinant of LAV/HTLV-III, and a physiologically acceptable carrier and/or diluent.

20. The pharmaceutical composition of claim 19 wherein said monoclonal antibody blocks the binding of an antibody produced by cell line LT1/41-H1 to said antigenic determinant.

21. The pharmaceutical composition of claim 19 wherein the monoclonal antibody binds an epitope on the envelope glycoprotein gp41.

22. The pharmaceutical composition of claim 19 wherein the monoclonal antibody is produced by the cell line LT1/41-H1.

23. A method for separating specific antigenic determinants of LAV/HTLV-III of interest from a mixture containing antigenic determinants of LAV/HTLV-III, a fraction of which contains the specific antigenic determinants, comprising:

immobilizing a human monoclonal antibody capable of reacting with the specific antigenic determinants on a substrate;

contacting the mixture containing the LAV/HTLV-III antigenic determinants with the immobilized antibody under suitable conditions such that immunecomplexes are formed between the antibody and the specific antigenic determinants; and

separating the immunecomplexes from the mixture.

24. The method of claim 23 wherein the monoclonal antibody blocks the binding of an antibody produced by cell line LT1/41-H1 to said antigenic determinant.

25. The method of claim 23 wherein the monoclonal antibody binds an epitope on the envelope glycoprotein gp41.

26. The method of claim 23 wherein the monoclonal antibody is produced by the cell line LT1/41-H1.

27. A composition comprising a human monoclonal antibody capable of reacting with an antigenic determinant of LAV/HTLV-III, and a physiologically acceptable carrier and/or diluent for use in reducing the infectivity of LAV/HTLV-III in warm blooded animals.

28. A method of producing a pharmaceutical composition comprising admixing a human monoclonal antibody capable of reacting with an antigenic determinant of LAV/HTLV-III, and a physiologically acceptable carrier and/or diluent.

29. A monoclonal antibody according to any one of Claims 1 to 5, or 9 (a) bound to a toxin, a radioactive material, a drug, or other conjugate, or (b), immobilized on a substrate.

30. A diagnostic kit comprising an antibody according to any one of Claims 1 to 5, 9, 10, or 29.

**Claims for the following Contracting States : ES, GR**

1. A method for determining the presence of LAV/HTLV-III in a biological sample comprising:
   incubating a human monoclonal antibody capable of reacting with an antigenic determinant of LAV/HTLV-III with a biological sample; and
   detecting the presence of immunecomplexes formed between said monoclonal antibody and said biological sample, and therefrom determining the presence of LAV/HTLV-III.

2. The method of any one of claim 1 wherein the monoclonal antibody is labelled.

3. The method of claim 2 wherein said label is selected from radionuclides, fluorescers, enzymes, enzyme substrates, enzyme cofactors, enzyme inhibitors, and ligands.

4. The method of any one of claims 1 to 3 wherein the step of detection is by enzyme reaction, fluorescence, radioactivity, cell lysis or luminescence emission.

5. The method of any one of claim 1 to 4 wherein the biological sample is selected from bodily secretions, bodily fluids and tissue specimens.

6. A method for separating specific antigenic determinants of LAV/HTLV-III of interest from a mixture containing antigenic determinants of LAV/HTLV-III, a fraction of which contains the specific antigenic determinants, comprising:
   immobilizing a human monoclonal antibody capable of reacting with the specific antigenic determinants on a substrate;
   contacting the mixture containing the LAV/HTLV-III antigenic determinants with the immobilized antibody under suitable conditions such that immunecomplexes are formed between the antibody and the specific antigenic determinants; and
   separating the immunecomplexes from the mixture.

7. The method of any one of claims 1 to 6 wherein said monoclonal antibody blocks the binding of an antibody produced by cell line LT1/41-H1 to said antigenic determinant.

8. The method of any one of claims 1 to 6 wherein the monoclonal antibody binds an epitope on the envelope glycoprotein gp41.

9. The method of any one of claims 1 to 6 wherein the monoclonal antibody is produced by the cell line LT1/41-H1.

10. A method of producing a pharmaceutical composition comprising admixing a human monoclonal antibody capable of reacting with an antigenic determinant of LAV/HTLV-III, and a physiologically acceptable carrier and/or diluent.

**Patentansprüche**
**Patentansprüche für folgende Vertragsstaaten : AT, BE, CH, DE, FR, GB, IT, LI, LU, NL, SE**

1. Menschlicher monoklonaler Antikörper, der zur Reaktion mit einer antigenen Determinante von LAV/HTLV-III fähig ist, worin der menschliche monoklonale Antikörper die Bindung eines von Zell-Linie LT1/41-H1 erzeugten Antikörpers an die antigene Determinante blockiert.

2. Menschlicher monoklonaler Antikörper, der ein Epitop auf dem Hüllenglycoprotein gp41 bindet.

3. Menschlicher monoklonaler Antikörper nach Anspruch 2, worin das Epitop vom durch pENV-3 kodierten Polypeptid definiert ist.

**4.** Menschlicher monoklonaler Antikörper nach Anspruch 2, worin das Epitop durch Peptid 39 definiert ist.

**5.** Menschlicher monoklonaler Antikörper nach Anspruch 2, worin das Epitop durch Peptid 79 definiert ist.

**6.** Immortalisierte Zell-Linie, die menschliche monoklonale Antikörper erzeugt, die zur Reaktion mit einer antigenen Determinante von LAV/HTLV-III fähig sind, worin die Zell-Linie ein Hybrid von menschlichen B-Lymphozyten ist, der zur Erzeugung von Antikörpern auf eine antigene Determinante von LAV/HTLV-III fähig ist, und eine Zelle ausgewählt aus der Gruppe von menschlichen Myelomzellen, Mäuse-Myelomzellen und menschlichen Lymphoblastoidzellen.

**7.** Immortalisierte Zell-Linie, die menschliche monoklonale Antikörper erzeugt, die zur Reaktion mit einer antigenen Determinante von LAV/HTLV-III fähig ist, worin die Zell-Linie B-Lymphozytenzellen aufweist, die zur Erzeugung von Antikörpern auf eine antigene Determinante von LAV/HTLV-III fähig ist, die mit Epstein-Barr-Virus transformierten Zellen transformiert sind.

**8.** Die Zell-Linie LT1/41-H1 (ATCC CRL-9128).

**9.** Monoklonaler Antikörper erzeugt durch die Zell-Linie nach Anspruch 8.

**10.** Menschlicher monoklonaler Antikörper nach einem der Ansprüche 1 bis 5 oder 9 versehen mit einem Marker, der fähig ist, ein nachweisbares Signal zu geben.

**11.** Verfahren zum Nachweis des Vorhandenseins von LAV/HTLV-III in einer biologischen Probe umfassend:
Inkubieren eines menschlichen monoklonalen Antikörpers, der zur Reaktion mit einer antigenen Determinante von LAV/HTLV-III fähig ist, mit einer biologischen Probe; und
Nachweis des Vorhandenseins von Immunkomplexen gebildet zwischen dem monoklonalen Antikörper und der biologischen Probe und daraus Nachweis des Vorhandenseins von LAV/HTLV-III.

**12.** Verfahren nach Anspruch 11, worin der monoklonale Antikörper die Bindung eines von Zell-Linie LT1/41-H1 erzeugten Antikörpers an die antigene Determinante blockiert.

**13.** Verfahren nach Anspruch 11, worin der monoklonale Antikörper ein Epitop auf dem Hüllenglycoprotein gp41 bindet.

**14.** Verfahren nach Anspruch 11, worin der monoklonale Antikörper von der Zell-Linie LT1/41-H1 erzeugt wird.

**15.** Verfahren nach einem der Ansprüche 11 bis 14, worin der monoklonale Antikörper markiert wird.

**16.** Verfahren nach Anspruch 15, worin der Marker ausgewählt ist aus der Gruppe von Radionukliden, fluoreszierenden Stoffen, Enzymen, Enzymsubstraten, Enzymcofaktoren, Enzyminhibitoren und Liganden.

**17.** Verfahren nach einem der Ansprüche 11 bis 16, worin der Nachweisschritt durch Enzymreaktion, Fluoreszenz, Radioaktivität, Zell-Lysis oder Lumineszenzemission durchgeführt wird.

**18.** Verfahren nach einem der Ansprüche 11 bis 17, worin die biologische Probe ausgewählt ist aus der Gruppe von Körperausscheidungen, Körperflüssigkeiten und Gewebeproben.

**19.** Pharmazeutische Zusammensetzung umfassend eine therapeutisch wirksame Menge eines menschlichen monoklonalen Antikörpers, der zur Reaktion mit einer antigenen Determinante von LAV/HTLV-III fähig ist, und einem physiologisch akzeptablen Träger und/oder Verdünnungsmittel.

**20.** Pharmazeutische Zusammensetzung nach Anspruch 19, worin der monoklonale Antikörper die Bindung eines durch die Zell-Linie LT1/41-H1 erzeugten Antikörpers an die antigene Determinante blockiert.

**21.** Pharmazeutische Zusammensetzung nach Anspruch 19, worin der monoklonale Antikörper ein Epitop auf dem Hüllenglycoprotein gp41 bindet.

**22.** Pharmazeutische Zusammensetzung nach Anspruch 19, worin der monoklonale Antikörper durch die Zell-Linie LT1/41-H1 erzeugt ist.

**23.** Verfahren zum Trennen spezifischer antigener Determinanten von LAV/HTLV-III von Interesse aus einer Mischung, die antigene Determinanten von LAV/HTLV-III enthält, von denen eine Fraktion die spezifischen antigenen Determinanten enthält, umfassend:

Immobilisieren eines menschlichen monoklonalen Antikörpers, der zur Reaktion mit den spezifischen antigenen Determinanten auf einem Substrat fähig ist;

in Kontakt bringen der Mischung, die die antigenen Determinanten von LAV/HTLV-III enthält, mit dem immobilisierten Antikörper unter geeigneten Bedingungen, in der Weise, daß Immunkomplexe zwischen dem Antikörper und den spezifischen antigenen Determinanten gebildet werden; und

Abtrennen der Immunkomplexe aus der Mischung.

**24.** Verfahren nach Anspruch 23, worin der monoklonale Antikörper die Bindung eines durch Zell-Linie LT1/41-H1 erzeugten Antikörpers an die antigene Determinante blockiert.

**25.** Verfahren nach Anspruch 23, worin der monoklonale Antikörper ein Epitop auf dem Hüllenglycoprotein gp41 bindet.

**26.** Verfahren nach Anspruch 23, worin der monoklonale Antikörper durch Zell-Linie LT1/41-H1 erzeugt wird.

**27.** Zusammensetzung umfassend einen menschlichen monoklonalen Antikörper, der zur Reaktion mit einer antigenen Determinante von LAV/HTLV-III fähig ist, und einen physiologisch akzeptablen Träger und/oder Verdünnungsmittel zur Verwendung beim Reduzieren der Infektionsfähigkeit von LAV/HTLV-III in warmblütigen Tieren.

**28.** Verfahren zur Herstellung einer pharmazeutischen Zusammensetzung umfassend Vermischen eines menschlichen monoklonalen Antikörpers, der zur Reaktion mit einer antigenen Determinante von LAV/HTLV-III fähig ist, und eines physiologisch akzeptablen Trägers und/oder Verdünnungsmittels.

**29.** Monoklonaler Antikörper nach einem der Ansprüche 1 bis 5 oder 9, (a) gebunden an ein Toxin, ein radioaktives Material, ein Arzneimittel oder ein anderes Konjugat oder (b) immobilisiert auf einem Substrat.

**30.** Diagnostischer Kit umfassend einen Antikörper nach einem der Ansprüche 1 bis 5, 9, 10 oder 29.

**Patentansprüche für folgende Vertragsstaaten : ES, GR**

**1.** Verfahren zum Nachweis des Vorhandenseins von LAV/HTLV-III in einer biologischen Probe umfassend:

Inkubieren eines menschlichen monoklonalen Antikörpers, der zur Reaktion mit einer antigenen Determinante von LAV/HTLV-III fähig ist, mit einer biologischen Probe; und

Nachweis des Vorhandenseins von Immunkomplexen gebildet zwischen dem monoklonalen Antikörper und der biologischen Probe und daraus Nachweis des Vorhandenseins von LAV/HTLV-III.

**2.** Verfahren nach Anspruch 1, worin der monoklonale Antikörper markiert wird.

**3.** Verfahren nach Anspruch 2, worin der Marker ausgewählt ist aus Radionukliden, fluoreszierenden Stoffen, Enzymen, Enzymsubstraten, Enzymcofaktoren, Enzyminhibitoren und Liganden.

**4.** Verfahren nach einem der Ansprüche 1 bis 3, worin der Nachweisschritt durch Enzymreaktion, Fluoreszenz, Radioaktivität, Zell-Lysis oder Lumineszenzemission durchgeführt wird.

**5.** Verfahren nach einem der Ansprüche 1 bis 4, worin die biologische Probe ausgewählt ist aus Körperausscheidungen, Körperflüssigkeiten und Gewebeproben.

**6.** Verfahren zum Trennen spezifischer antigener Determinanten von LAV/HTLV-III von Interesse aus einer Mischung, die antigene Determinanten von LAV/HTLV-III enthält, von denen eine Fraktion die spezifischen antigenen Determinanten enthält, umfassend:

Immobilisieren eines menschlichen monoklonalen Antikörpers, der zur Reaktion mit den spezifischen antigenen Determinanten auf einem Substrat fähig ist;

in Kontakt bringen der Mischung, die die antigenen Determinanten von LAV/HTLV-III enthält, mit dem immobilisierten Antikörper unter geeigneten Bedingungen, in der Weise, daß Immunkomplexe zwischen dem Antikörper und den spezifischen antigenen Determinanten gebildet werden; und

Abtrennen der Immunkomplexe aus der Mischung.

**7.** Verfahren nach einem der Ansprüche 1 bis 6, worin der monoklonale Antikörper die Bindung eines durch Zell-Linie LT1/41-H1 erzeugten Antikörpers an die antigene Determinante blockiert.

**8.** Verfahren nach einem der Ansprüche 1 bis 6, worin der monoklonale Antikörper ein Epitop auf dem Hüllenglycoprotein gp41 bindet.

**9.** Verfahren nach einem der Ansprüche 1 bis 6, worin der monoklonale Antikörper durch Zell-Linie LT1/41-H1 erzeugt wird.

**10.** Verfahren zur Herstellung einer pharmazeutischen Zusammensetzung umfassend Vermischen eines menschlichen monoklonalen Antikörpers, der zur Reaktion mit einer antigenen Determinante von LAV/HTLV-III fähig ist, und eines physiologisch akzeptablen Trägers und/oder Verdünnungsmittels.

**Revendications**
**Revendications pour les Etats contractants suivants : AT, BE, CH, DE, FR, GB, IT, LI, LU, NL, SE**

**1.** Anticorps monoclonal humain capable de réagir avec un déterminant antigénique de LAV/HTLV-III, où l'anticorps monoclonal humain bloque la liaison d'un anticorps produit par la lignée de cellules LT1/41-H1 audit déterminant antigénique.

**2.** Anticorps monoclonal humain qui lie un épitope sur la glycoprotéine gp41 de l'enveloppe.

**3.** Anticorps monoclonal humain de la revendication 2, où l'épitope est défini par le polypeptide codé par pENV-3.

**4.** Anticorps monoclonal humain de la revendication 2, où l'épitope est défini par le peptide 39.

**5.** Anticorps monoclonal humain de la revendication 2, où l'épitope est défini par le peptide 79.

**6.** Lignée de cellules immortalisées qui produit un anticorps monoclonal humain capable de réagir avec un déterminant antigénique de LAV/HTLV-III, où la lignée de cellules est un hybride de lymphocytes B humains capables de produire des anticorps à un déterminant antigénique de LAV/HTLV-III et d'une cellule sélectionnée dans le groupe consistant en cellules de myélome humain, de cellules de myélome de souris et de cellules de lymphoblastoïde humain.

**7.** Lignée de cellules immortalisées qui produit l'anticorps monoclonal humain capable de réagir avec un déterminant antigénique de LAV/HTLV-III, où la lignée de cellules comprend des cellules de lymphocytes B capables de produire des anticorps à un déterminant antigénique de LAV/HTLV-III, transformées par des cellules transformées par le virus de Epstein-Barr.

**8.** Lignée de cellules LT1/41-H1 (ATCC CRL-9128).

**9.** Anticorps monoclonal produit par la lignée de cellules de la revendication 8.

**10.** Anticorps monoclonal humain selon l'une quelconque des revendications 1 à 5 ou 9, marqué d'un marqueur capable de produire un signal détectable.

**11.** Méthode de détermination de la présence de LAV/HTLV-III dans un échantillon biologique comprenant :

l'incubation d'un anticorps monoclonal humain capable de réagir avec un déterminant antigénique de LAV/HTLV-III avec un échantillon biologique , et

la détection de la présence d'immunocomplexes formés entre ledit anticorps monoclonal et ledit échantillon biologique et la détermination, à partir de cela, de la présence de LAV/HTLV-III.

**12.** Méthode de la revendication 11, où ledit anticorps monoclonal bloque la liaison d'un anticorps produit par la lignée de cellules LT1/41-H1 audit déterminant antigénique.

**13.** Méthode de la revendication 11, où l'anticorps monoclonal lie un épitope sur la glycoprotéine gp41 de l'enveloppe.

**14.** Méthode de la revendication 11, où l'anticorps monoclonal est produit par la lignée de cellules LT1/41-H1.

**15.** Méthode selon l'une quelconque des revendications 11 à 14, où l'anticorps monoclonal est marqué.

**16.** Méthode de la revendication 15, où ledit marqueur est choisi dans le groupe consistant en radionuclibles, agents fluorescents, enzymes, substrats d'enzyme, cofacteurs d'enzyme, inhibiteurs d'enzyme et ligands.

**17.** Méthode selon l'une quelconque dee revendications 11 à 16, où l'étape de détection est par réaction d'enzyme, fluorescence, radioactivité, lyse de cellules ou émission de luminescence.

**18.** Méthode selon l'une quelconque des revendications 11 à 17, où l'échantillon biologique est choisi dans le groupe consistant en sécrétions corporelles, fluides corporels et spécimens de tissu.

**19.** Composition pharmaceutique comprenant une quantité thérapeutiquement efficace d'un anticorps monoclonal humain capable de réagir avec un déterminant antigénique de LAV/HTLV-III et un support et/ou diluant physiologiquement acceptables.

**20.** Composition pharmaceutique de la revendication 19, où ledit anticorps monoclonal bloque la liaison d'un anticorps produit par la lignée de cellules LT1/41-H1 audit déterminant antigénique.

**21.** Composition pharmaceutique de la revendication 19, où l'anticorps monoclonal lie un épitope sur la glycoprotéine gp41 de l'enveloppe.

**22.** Composition pharmaceutique de la revendication 19, où l'anticorps monoclonal est produit par la lignée de cellules LT1 41-H1.

**23.** Méthode de séparation de déterminants antigéniques spécifiques de LAV/HTLV-III d'intérêt d'un mélange contenant des déterminants antigéniques de LAV/HTLV-III, dont une fraction contient les déterminants antigéniques spécifiques,consistant à :

immobiliser un anticorps monoclonal humain capable de réagir avec les déterminants antigéniques spécifiques sur un substrat ;

mettre le mélange contenant les déterminants antigéniques de LAV/HTLV-III en contact avec l'anticorps immobilisé en conditions appropriées de manière que des immunocomplexes se forment entre l'anticorps et les déterminants antigéniques spécifiques ; et

séparer les immunocomplexes du mélange.

**24.** Méthode de la revendication 23, où l'anticorps monoclonal bloque la liaison d'un anticorps produit par la lignée de cellules LT1/41-H1 audit déterminant antigénique.

**25.** Méthode de la revendication 23, où l'anticorps monoclonal lie un épitope sur la glycoprotéine gp41 de l'enveloppe.

EP 0 251 612 B1

26. Méthode de la revendication 23, où l'anticorps monoclonal est produit par la lignée de cellules LT1/41-H1.

27. Composition comprenant un anticorps monoclonal humain capable de réagir avec un déterminant antigénique de LAV/HTLV-III et un support et/ou diluant physiologiquement acceptables pour une utilisation pour la réduction de l'infectivité de LAV/HTLV-III chez des animaux à sang chaud.

28. Méthode de production d'une composition pharmaceutique consistant à mélanger un anticorps monoclonal humain capable de réagir avec un déterminant antigénique de LAV/HTLV-III et un support et/ou diluant acceptables physiologiquement.

29. Anticorps monoclonal selon l'une quelconque des revendications 1 à 5 ou 9 (a) lié à une toxine, une matière radioactive, un médicament ou autre conjugé ou (b), immobilisé sur un substrat.

30. Nécessaire de diagnostic comprenant un anticorps selon l'une quelconque des revendications 1 à 5, 9, 10 ou 29.

**Revendications pour les Etats contractants suivants : ES, GR**

1. Méthode de détermination de la présence de LAV/HTLV-III dans un échantillon biologique, comprenant :

   l'incubation d'un anticorps monoclonal humain capable de réagir avec un déterminant antigénique de LAV/HTLV-III avec un échantillon biologique ; et
   la détection de la présence d'immunocomplexes formés entre ledit anticorps monoclonal et ledit échantillon biologique et la détermination, à partir de cela, de la présence de LAV/HTLV-III.

2. Méthode selon la revendication 1, où l'anticorps monoclonal est marqué.

3. Méthode de la revendication 2, où ledit marqueur est choisi parmi des radionuclides, agents fluorescents, enzymes, substrats d'enzyme, cofacteurs d'enzyme, inhibiteurs d'enzyme et ligands.

4. Méthode selon l'une quelconque des revendications 1 à 3, où l'étape de détection est par réaction enzymatique, fluorescence, radioactivité, lyse de cellules ou émission de luminescence.

5. Méthode selon l'une quelconque des revendications 1 à 4, où l'échantillon biologique est choisi parmi des sécrétions corporelles, des fluides corporels et des spécimens de tissu.

6. Méthode de séparation de déterminants antigéniques spécifiques de LAV/HTLV-III d'intérêt, d'un mélange contenant des déterminants antigéniques de LAV/HTLV-III dont une fraction contient les déterminants antigéniques spécifiques, consistant à :
   immobiliser un anticorps monoclonal humain capable de réagir avec les déterminants antigéniques spécifiques sur un substrat ;
   mettre le mélange contenant les déterminants antigéniques de LAV/HTLV-III en contact avec l'anticorps immobilisé en conditions appropriées de manière que des immunocomplexes se forment entre l'anticorps et les déterminants antigéniques spécifiques ; et
   séparer les immunocomplexes du mélange.

7. Méthode selon l'une quelconque des revendications 1 à 6, où ledit anticorps monoclonal bloque la liaison d'un anticorps produit par la lignée de cellules LT1/41-H1 audit déterminant antigénique.

8. Méthode selon l'une quelconque des revendications 1 à 6, où l'anticorps monoclonal lie un épitope sur la glycoprotéine gp41 de l'enveloppe.

9. Méthode selon l'une quelconque des revendications 1 à 6, où l'anticorps monoclonal est produit par la lignée de cellules LT1/41-H1.

10. Méthode de production d'une composition pharmaceutique, consistant à mélanger un anticorps monoclonal humain capable de réagir avec un déterminant antigénique de LAV/HTLV-III et son support

17

et/ou diluant physiologiquement acceptable.

Fig.1

**FIGURE 2**

ENV-3

_____ #5 _____

Ile Phe Arg Pro Gly Gly Gly Asp Met Arg Asp Asn Trp Arg Ser Glu

                                                    30
Leu Try Lys Tyr Lys Val Val Lys Ile Glu Pro Leu Gly Val Ala Pro

_____ #2 _____ 40 _____
Thr Lys Ala Lys Arg Arg Val Val Gln Arg Glu Lys Arg Ala Val Gly

_____ 50 _____ #1 _____          60
Ile Gly Ala Leu Phe Leu Gly Phe Leu Gly Ala Ala Gly Ser Thr Met

                70                                80
Gly Ala Gly Ser Met Thr Leu Thr Val Gln Ala Arg Gln Leu Leu Ser

                        90
Gly Ile Val Gln Gln Gln Asn Asn Leu Leu Arg Ala Ile Glu Ala Gln

        100                                110
Gln His Leu Leu Gln Leu Thr Val Trp Gly Ile Lys Gln Leu Gln Ala

                        120
Arg Ile Leu Ala Val Glu Arg Tyr Leu Lys Asp Gln Gln Leu Leu Gly

    130 _____ #3 _____ 140
Ile Trp Gly Cys Ser Gly Lys Leu Ile Cys Thr Thr Ala Val Pro Trp

                150                                160
Asn Ala Ser Trp Ser Asn Lys Ser Leu Glu Gln Ile Trp Asn Asn Met

                        170   #4 _____
Thr Trp Met Glu Trp Asp Arg Glu Ile Asn Asn Tyr Thr